# EUROPEAN PATENT SPECIFICATION

(11) **EP 2 207 502 B1**
(45) Date of publication and mention of the grant of the patent: **09.03.2011**
(21) Application number: 08846546.3
(22) Date of filing: 21.10.2008
(51) Int. Cl.: A61F 2/16, G02B 1/04

(54) **PHOTOREFRACTIVE MULTI-ELEMENT OPHTHALMIC LENS**
MEHRTEILIGE PHOTOREFRAKTIVE OPHTHALMISCHE LINSE
LENTILLE OPHTALMIQUE PHOTOREFRACTIVE À MULTIPLES ÉLÉMENTS

(30) Priority: 06.11.2007 US 935506
(43) Date of publication of application: 21.07.2010
(73) Proprietor: Bausch & Lomb Incorporated, Rochester, NY 14604-2701 (US)
(72) Inventor: ALTMANN, Griffith, E., Pittsford, NY 14534 (US)
(74) Representative: Vossius & Partner
(86) International application number: PCT/US2008/080632
(87) International publication number: WO 2009/061606

(56) References cited:
- US-A1- 2004 015 236
- US-A1- 2007 055 369
- US-B1- 6 450 642

## Description

### Field of Invention

The present invention relates to multi-element ophthalmic lenses (OLs), and more particularly to light-adjustable, multi-element ophthalmic lenses.

### Background of the Invention

In an eye where the natural crystalline lens has been damaged (e.g., clouded by cataracts), the natural lens is no longer able to properly focus and/or direct incoming light to the retina. As a result images become blurred. A well known surgical technique to remedy this situation involves removal of a damaged crystalline lens through a hole in the capsular bag known as a capsularhexis (also referred to simply as a rhexis). After removal, an artificial lens known as an intraocular lens (IOL) can be placed into the evacuated capsular bag through the rhexis.

In theory, the optical power of IOLs that is required for emmetropia (i.e., point focus on the retina for light originating at infinity) can be precisely calculated. The power of the implanted lens is calculated based on pre-operative measurements of one or more of ocular lengths and corneal curvatures. Unfortunately, due to errors in measurement, imprecise lens positioning or unpredicted wound healing, most patients undergoing cataract surgery will not enjoy optimal vision without some form of vision correction following the surgery (Brandser et al., Acta Opthalmol Scand 75:162 165 (1997); Oshika et al., J Cataract Refract Surg 24:509 514 (1998). Because the power of most conventional IOLs cannot be adjusted post-implantation, patients typically require additional corrective lenses such as eye glasses or contact lenses.

One proposed solution to the foregoing problem is a light-adjustable intraocular lens whose refraction properties can be modified following insertion of the lens into a human eye. Such a lens is described in U.S. Patent Application 11/745,746 titled OPTICAL MATERIAL AND METHOD FOR MODIFYING THE REFRACTIVE INDEX, filed May 8, 2007 by inventors Kunzler, et al. The substance of said application is hereby incorporated by reference in its entirety. In said application, some embodiments are described in which relatively short wavelengths of light are projected onto a photopolymerizable material constituting the lens, thereby changing the index of refraction, and as a result the optical power of the lens.

Another light-adjustable lens is reported in U.S. Patent No. 6,450,642, hereafter referred to as the Jethmalani Patent. Said patent discloses apparatus and techniques indicated to result in a light-adjusted variable-power lens in which the power of the lens is changed by a shape-change arising due to the migration (i.e., diffusion) of a photopolymerizable material within another polymer matrix. The power change is achieved during multiple radiation exposures and diffusion periods.

A modification to light-adjustable lenses (such as those described above) in which a lens is coated with an ultraviolet light-blocking layer that is disposed on a surface of the lens is known. PCT Patent Application WO2007030799 to Chang describes a lens made of light-adjustable material and having a UV-blocking layer disposed on a posterior surface of the lens. The lens is indicated to permit the use of a higher intensity of radiation to polymerize the photopolymerizable material while protecting a subject's eye from the UV radiation.

### Summary

Conventional IOLs such as those described above are single-element, non-accommodative lenses. Such lenses are usually selected to have a power such that the patient has a fixed-focus for distance vision, and the patient requires spectacles or contact lenses to permit near vision.

Multi-element OLs are also known. Such lenses may comprise two or more lens elements. One example of a multi-element OL is a dual element OL.

Multi-element OLs comprise an anterior lens element and a posterior element. The term "anterior lens" refers to a lens that is designed to be placed relatively nearer the cornea of an eye; and the term "posterior lens" refers to a lens that is designed to be placed nearer the retina of the eye. The terms "anterior lens" and "posterior lens" are not limited to lenses in a system that are closest to the cornea or retina (i.e., in a three lens system any two lens may constitute an anterior lens/posterior lens pair); rather, these terms are used to describe relative position.

Multi-element OLs may be accommodative or non-accommodative. In recent years, extensive research has been carried out to develop accommodative IOLs that have a variable focus that varies in response to the accommodative actions of the eye (e.g., stretching or relaxing of the zonules of the eye) or other mechanical actuation. Such IOLs are known as accommodative IOLs (AIOLs). Multi-element AIOLs are designed to provide variable focus due to translation of one or more of the optics and/or due to changes in the shape of the lens in response to the accommodative actions.

Aspects of the present invention are directed to multi-element OLs comprising an anterior lens element comprising a light-adjustable material, and a posterior lens element comprising a light blocker adapted to block light capable of producing a power change in the light-adjustable anterior optic. Accordingly, light that is capable of producing a power change can be projected into the eye and into the anterior optic to attain an adjustment of the optical power of IOL (in particular, the power of the anterior lens element), while the posterior lens blocks said light, thereby protecting the posterior portions of eye (e.g. the retina) from damage arising from the exposure to the light.

A first aspect of the present invention is directed to a multi-element OL, comprising an anterior lens element comprising a light-adjustable material responsive to light of a first wavelength, and a posterior lens element comprising a light blocker capable of blocking light of the first wavelength.

In some embodiment, the OL is an accommodative IOL. In some embodiments, the anterior lens element and the posterior lens element are mechanically connected together. In some embodiments, the light-adjustable material is responsive to light in the range 300 nm to 450 nm.

In some embodiments, the anterior lens element is adapted to be located in an anterior chamber of an eye and the posterior lens element is adapted to be located in a posterior chamber of the eye.

Another aspect of the invention is directed to a method of implanting a multi-element OL, comprising implanting a first lens element comprising a light-adjustable material responsive to light of a first wavelength, and implanting a second lens element posterior to the first lens element, the second lens element comprising a light blocker capable of blocking light of the first wavelength.

In some embodiments of the method, the first lens element and second lens element are mechanically coupled together. In some embodiments, the method further comprises projecting light of the first wavelength onto the second element by projecting the light through the first element.

In some embodiments, the step of implanting the first lens element comprises implanting the first lens element in an anterior portion of the eye. In such embodiments, the step of implanting the second lens element may comprise implanting the first lens element in a posterior chamber of the eye.

In some embodiments, the step of implanting the first lens element comprises implanting the first lens element in a posterior chamber of the eye and the step of implanting the second lens element comprises implanting the second lens element in the posterior chamber of the eye.

In some embodiments, the step of implanting the first lens element comprises implanting the first lens element in an anterior portion of the eye and the step of implanting the second lens element comprises implanting the second lens element in the anterior portion of the eye.

In some embodiments, the step of implanting the second lens element is performed prior to the step of implanting the first lens element.

The term "ophthalmic lens" as used herein is defined to mean any lens adapted to be implanted in an eye. For example, an ophthalmic lens may comprise a corneal inlay or onlay, a anterior chamber IOL or a posterior chamber IOL.

### Brief Description of the Drawings

Illustrative, non-limiting embodiments of the present invention will be described by way of example with reference to the accompanying drawings, in which the same reference number is used to designate the same or similar components in different figures, and in which:
FIG. 1 is a schematic illustration of a dual-element IOL;
FIGs. 2A and 2B schematically illustrate an example of molding apparatus according to aspects of the present invention, in disassembled and assembled states, respectively.

### Detailed Description

Aspects of the invention are directed to multi-element ophthalmic lenses comprising an anterior lens element including a light-adjustable material responsive to light of a first wavelength and a posterior lens element comprising a light blocker capable of blocking light of the first wavelength.

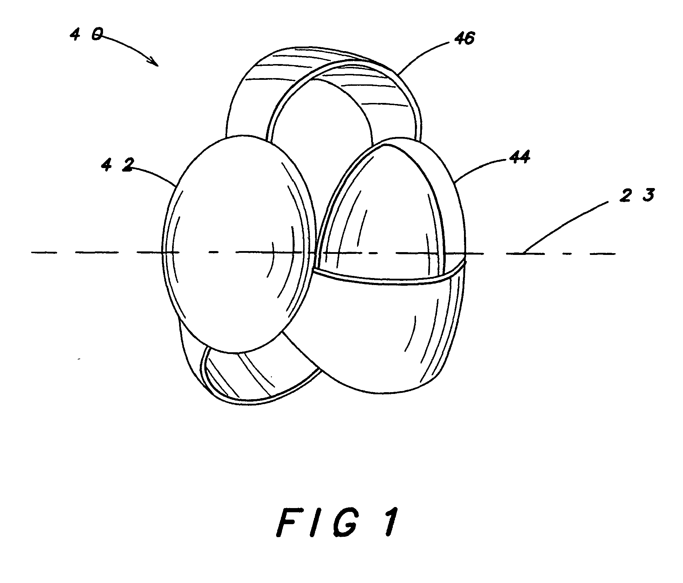

FIG. 1 shows an example of a dual-element IOL 40 according to aspects of the invention. IOL 40 comprises an anterior lens element 42 and a posterior lens element 44 that are connected to one another by biasing elements 46 (e.g., haptics). The IOL has an optical axis 23 extending through the anterior lens element 42 and posterior lens element 44. The anterior lens element comprises a light-adjustable material, and the posterior lens element comprises an ultraviolet (UV) light blocker.

Biasing elements 46 permit the anterior lens element and the posterior lens element to translate relative to one another to achieve accommodation and disaccommodation, and provide centering of the lens within the capsular bag. Further details of IOL 40 are given in U.S. Pat. No. 6,488,708 issued December 3, 2002, to Sarfarazi, and an alternative configuration is given in U.S. Pat. No. 6,761,737 issued July 13, 2004, to Zadno-Azizi, et al. Both of said patents are hereby incorporated by reference in therein entirety.

As noted above, the anterior lens element 42 comprises a light-adjustable material. The light-adjustable material may comprise any suitable material capable of changing optical power of a lens which it constitutes, in response to exposure to light of a given wavelength or a band of wavelengths including a given wavelength. For example, the anterior optic may comprise an acrylic or silicone material containing materials capable of changing the index of refraction or shape in response to light incident thereon as described in the Background section above. It will be appreciated that exposure to the light may also be used to adjust (i.e., reduce or increase) aberrations of a wavefront passing through the lens. The term "light" as used herein, for example as used in the term "light-adjustable" includes radiation in the ultraviolet range or any other suitable range. Typically, light capable of adjusting a lens element is outside of the visible range, but may extend into a portion of the visible range.

As noted above, the posterior lens element 44 comprises a light blocker. The light blocker is capable of blocking light of the given wavelength that is capable of changing the optical power of the anterior lens element or, in the event that the anterior lens is to be exposed to light in a band of wavelengths, the light blocker will preferably be capable of blocking light in a wavelength band. Preferably the light blocker does not substantially affect the transmission of light within the visible spectrum of the patient's in which the lenses are to be inserted. However, as stated above, light capable of adjusting a lens element may extend into the visible range, and it may be desirable to substantially completely or at least partially block such light. Typically, the light-adjustable lens element will be responsive to light in the range 300 nm to 450 nm, and the posterior lens will block light in said range and perhaps at least some light having shorter wavelengths. Examples of suitable UV-blocker materials are given in U.S. Patent No. 4,803,254, issued February 7, 1989 to Dunks, et al. and 4,716,234 issued December 29, 1987. Both of said patents are hereby incorporated by reference in their entirety.

Although the above embodiment is an AIOL, aspects of the present invention are directed to multi-element IOLs regardless of whether they are accommodative or non-accommodative. Additionally, regardless of whether the lens is accommodative, the anterior lens element and the posterior lens element may be mechanically coupled together or not. For example, in some embodiments, the anterior lens is adapted to be located in an anterior chamber of an eye or the lens is a corneal inlay or onlay, and the posterior lens is adapted to be located in the posterior chamber of the eye (e.g., in a capsular bag). As used herein the posterior chamber includes any portion of the eye behind the iris. Alternatively, both lenses may be located in the posterior chamber of the eye (e.g., the anterior lens is adapted to be located in the ciliary sulcus and the posterior lens is adapted to be located in the capsular bag). In yet another alternative, both lenses may be located in anterior portions of the eye (e.g., the anterior lens may be a corneal inlay or onlay and the posterior lens may be located in the anterior chamber). The term "anterior portion of the eye" refers to portions in front of the iris.

Manufacture of the elements comprising the lens may be achieved using any suitable technique. Regardless of whether the lenses are to be mechanically coupled or not, the lenses may be made using any suitable machining and/or molding techniques. Lenses to be mechanically coupled together may be manufactured as an integrated unit (including biasing elements extending between a first lens element and a second lens element). For example, the lenses may be made using liquid injection molding techniques. One example of an apparatus for molding a lens comprising integrated optics and haptics extending therebetween is given in U.S. Application No. 10/ 954,322 titled APPARATUS AND METHOD FOR INJECTION MOLDING AN INTRAOCULAR LENS DEVICE, filed September 30, 2004, by Graney et al. (hereinafter Graney). The substance of said application in said application is hereby incorporated by reference. The apparatus disclosed in said application comprises three mold components: a first cavity block for forming one optical surface of a first lens element and a second cavity block for forming a first surface of a second lens element, and an insert that fits between the first block and the second block and forms a second (interior) surface of the first lens element and a second (interior) surface of the second lens element. It is apparent that such apparatus form a first cavity in which the first lens element is formed and a second cavity in which the second lens element is formed.

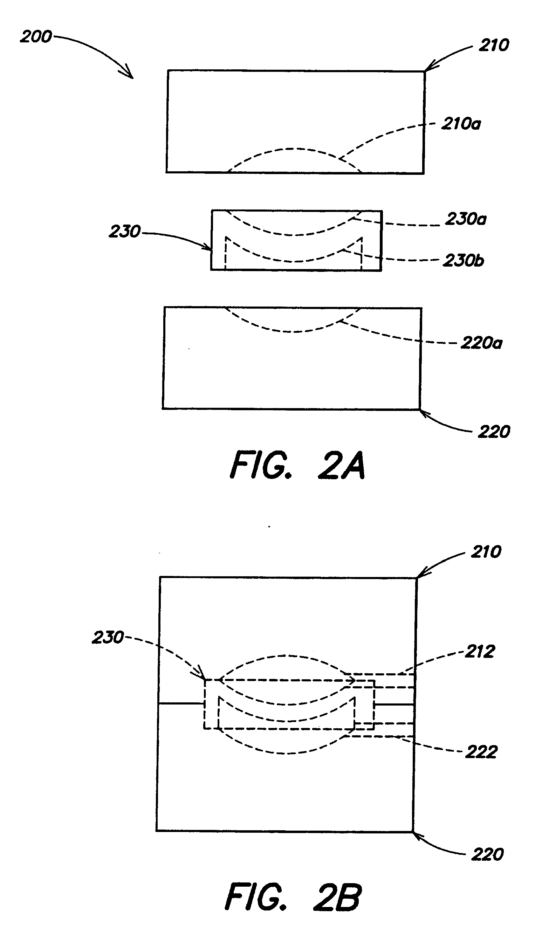

FIGs. 2A and 2B schematically illustrate an example of molding apparatus 200 according to aspects of the present invention, in disassembled and assembled states respectively. Similar to the apparatus of Graney, apparatus 200 includes three mold components: a first cavity block 210 for forming one optical surface 210a of a first lens element, a second cavity block 220 for forming a first surface 220a of a second lens element, and an insert 230 that fits between the first block and the second block and forms a first (interior) surface 230a of the first lens element and a first (interior) surface 230b of the second lens element; however, in contrast to the apparatus of Graney, the apparatus in FIGs. 2A and 2B comprises a first runner 212 through which material containing light-adjustable material to form the anterior lens element can be injected into a first cavity that is separate of a second runner 222, leading to a second cavity, through which material containing light-blocking material to form the posterior lens element is injected. The separate runners may be completely separate of one another or may be separated by one or more valves. In the event that one or more valves are included, the runners may have one or more runner portions that are used for delivery of material to both the first cavity and the second cavity.

Having thus described the inventive concepts and a number of exemplary embodiments, it will be apparent to those skilled in the art that the invention may be implemented in various ways, and that modifications and improvements will readily occur to such persons. Thus, the embodiments are not intended to be limiting and presented by way of example only. The invention is limited only as required by the following claims.

## Claims

1. A multi-element ophthalmic lens (OL), comprising:
an anterior lens element comprising a light-adjustable material responsive to light of a first wavelength; and
a posterior lens element comprising a light blocker capable of blocking light of the first wavelength.

2. The OL of claim 1, wherein the OL is an accommodative IOL.

3. The OL of claim 1, wherein the anterior lens element and the posterior lens element are mechanically connected together.

4. The OL of claim 1, wherein the light-adjustable material is responsive to light having a wavelength in the range 300 nm to 450 nm.

5. The OL of claim 1, wherein anterior lens element is adapted to be located in an anterior chamber of an eye and the posterior lens element is adapted to be located in a posterior chamber of the eye.

## Patentansprüche

1. Mehrelementige ophthalmische Linse (OL) mit:
einem vorderen Linsenelement mit einem lichteinstellbaren Material, das auf Licht mit einer ersten Wellenlänge reagiert; und
einem hinteren Linsenelement mit einem Lichtblocker, der Licht mit der ersten Wellenlänge blockieren kann.

2. OL nach Anspruch 1, wobei die OL eine akkommodative IOL ist.

3. OL nach Anspruch 1, wobei das vordere Linsenelement und das hintere Linsenelement mechanisch miteinander verbunden sind.

4. OL nach Anspruch 1, wobei das lichteinstellbare Material auf Licht mit einer Wellenlänge im Bereich von 300 nm bis 450 nm reagiert.

5. OL nach Anspruch 1, wobei das vordere Linsenelement geeignet ist, in einer Vorderkammer eines Auges angeordnet zu sein, und das hintere Linsenelement geeignet ist, in einer Hinterkammer des Auges angeordnet zu sein.

## Revendications

1. Lentille ophtalmique (OL) à éléments multiples comprenant :
un élément de lentille antérieur comprenant un matériau pouvant s'ajuster à la lumière, sensible à la lumière d'une première longueur d'onde ; et
un élément de lentille postérieur comprenant un bloqueur de lumière, capable de bloquer la lumière de la première longueur d'onde.

2. OL selon la revendication 1, laquelle OL est une IOL accommodative.

3. OL selon la revendication 1, dans laquelle l'élément de lentille antérieur et l'élément de lentille postérieur sont connectés ensemble mécaniquement.

4. OL selon la revendication 1, dans laquelle le matériau pouvant s'ajuster à la lumière est sensible à la lumière ayant une longueur d'onde située dans la plage allant de 300 nm à 450 nm.

5. OL selon la revendication 1, dans laquelle l'élément de lentille antérieur est adapté pour être localisé dans une chambre antérieure d'un oeil et l'élément de lentille postérieur est adapté pour être localisé dans une chambre postérieure de l'oeil.
